(19) Europäisches Patentamt
European Patent Office
Office européen des brevets

(11) Publication number: **0 016 590**
**B1**

(12) # EUROPEAN PATENT SPECIFICATION

(45) Date of publication of patent specification: **06.06.84**

(51) Int. Cl.³: **C 07 C 145/00,**
C 07 D 307/86,
A 01 N 47/10//C07D317/64

(21) Application number: **80300691.5**

(22) Date of filing: **06.03.80**

(54) **N-Sulfinyl carbamates, their use as pesticides, pesticidal compositions containing them, methods of producing them and halosulfinyl carbamates formed during their production.**

(30) Priority: **07.03.79 US 18414**
**07.03.79 US 18416**
**07.03.79 US 18417**
**07.03.79 US 18598**
**02.04.79 US 26364**

(43) Date of publication of application:
**01.10.80 Bulletin 80/20**

(45) Publication of the grant of the patent:
**06.06.84 Bulletin 84/23**

(84) Designated Contracting States:
**AT BE CH DE FR GB IT LU NL SE**

(56) References cited:
**BE - A - 848 913**
**GB - A - 1 486 969**
**US - A - 3 843 689**

**K.H. Büchel: "Pflanzenschutz u. Schädlingsbekämpfung" (1977) G. Thieme, Stuttgart; p. 71, nr. 40,41**

**J.Agr. Food Chem, 28, 1325-1330 (1980)**

**J.C. Street: "Pesticide Selectivity" (1975) M. Dekker, N.Y., p. 100-4**

**The file contains technical information submitted after the application was filed and not included in this specification**

(73) Proprietor: **THE REGENTS OF THE UNIVERSITY OF CALIFORNIA**
**2199 Addison Street**
**Berkeley California 94720 (US)**

(72) Inventor: **Fahmy, Mohamed Abdel Hamid**
**16-A Grandview Avenue**
**Edison New Jersey 08817 (US)**
Inventor: **Fukuto, Tetsuo Roy**
**144 East Broadbent Drive**
**Riverside, California 92507 (US)**

(74) Representative: **Crampton, Keith John Allen et al,**
**D YOUNG & CO 10 Staple Inn**
**London WC1V 7RD (GB)**

(56) References cited:

**Pesticide Biochemistry and Physiology 3, p. 435-446(1973)**

**J. Agr. and Food Chem. Vol. 21, 6, p. 1037-47 (1973)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 016 590 B1**

## Description

The present invention is directed to a new class of carbamate pesticides, to intermediates therefor, to an insecticidal method and composition thereof, and to a process for manufacture. More particularly, the invention is directed to N-sulfinyl carbamates useful as insecticides or insecticide intermediates.

Various types of carbamate pesticides have been previously described. For example, U.S. Patent No. US—A—3,997,549 discloses N-arylsulferylated derivatives of benzofuranyl methylcarbamates as effective pesticides; U.S. Patent No. US—A—4,006,231 discloses N-aminosulfenylated derivatives of carbofuran as effective pesticides; and U.S. Patent No. US—A—3,843,689 discloses production of N-methyl or N-phenyldithiocarbamates produced from N-chlorothiocarbamates, as insecticides. Other pesticidal N-sulfenyl carbonates are disclosed in United Kingdom Patent No. GB—A—1,486,969, in Belgian Patent No. BE—A—848913 and in K. H. Büchel: "Pflanzenschutz u. Schädlingsbekämfung" (1977) G. Thieme, Stuttgart; (see page 71, Nos. 40 and 41). Insecticidal N-sulfinylcarbamates have been described in: Pesticide Biochemistry and Physiology; *3*, p. 435—446 (1973) and Journal of Agricultural Food Chemistry: Vol. 21, *6*, p. 1037—1047. (1973).

The insecticidal compounds of this invention have a high level of insecticidal activity and improved mammalian toxicity. Activity is generally about equal to that for the carbamate insecticide from which the compound is derived, but mammalian toxicity is generally substantially lower.

The sulfinylcarbamates of this invention are those having the formula:

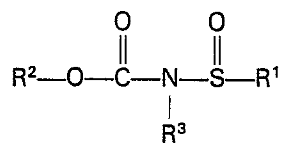

I

in which:

$R^1$ is bromine, chlorine or fluorine, preferably chlorine;

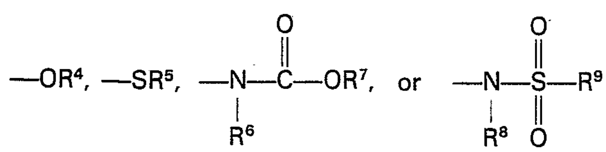

$R^2$ and $R^7$, the same or different, are alkyl of 1 to 8 carbon atoms; naphthyl; phenyl a substituted aryl group of the formula

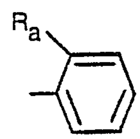

or

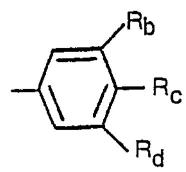

wherein

$R_a$ is alkyl, alkoxy, alkylthio, alkylthioalkyl or 2-dioxolanyl,

$R_b$ is hydrogen, alkyl, alkoxy, alkoxyalkyl, formyl(alkyl)amino, or halogen (chlorine or bromine),

$R_c$ is hydrogen, alkyl, alkoxy, alkylthio or dialkylamino,

$R_d$ is hydrogen or alkyl,

and each alkyl group contains 1 to 6, preferably 1 to 4 carbon atoms;

a heterocyclic group of the formula

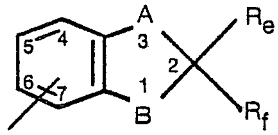

in which $R_e$ and $R_f$ are hydrogen or alkyl of 1 to 4 carbon atoms, A and B are each oxygen or one of A and B is methylene and the other is oxygen or sulfur, attached at the 4 or 7 position, for example, 2,3-dihydro-2,2-dimethyl-7-benzofuranyl or 2,2-dimethyl-1,3-benzodioxol-4-yl; or

an imino group of the formula $R_gR_hC{=}N—$ wherein $R_g$ is hydrogen or alkyl of 1 to 4 carbon atoms and $R_h$ is alkylthio, alkylthioalkyl or dialkylaminocarbonyl, in which the alkyl groups, the same or different, have 1 to 4 carbon atoms;

$R^3$, $R^6$ and $R^8$, the same or different, are alkyl of 1 to 4 carbon atoms or phenyl optionally substituted with alkyl or halogen;

$R^4$ and $R^5$ are alkyl of 1 to 12 carbon atoms, preferably 3 to 10 carbon atoms; unsaturated aliphatic hydrocarbon of 2 to 4 carbon atoms; or phenyl optionally substituted with alkyl of 1 to 6 carbon atoms, alkylthio of 1 to 4 carbon atoms or di($C^{1-4}$)alkylamino;

$R^9$ is alkyl of 1 to 4 carbon atoms; phenyl optionally substituted with alkyl or halogen; or the group —$NR^{10}R^{11}$, in which $R^{10}$ and $R^{11}$ are alkyl of 1 to 6 carbons or taken together with the nitrogen form a morpholino, piperidyl, or pyridyl ring.

At least one of the substituents $R^2$ or $R^7$ must be other than phenyl (unsubstituted) or alkyl, and the substituent on the corresponding carbamate nitrogen ($R^3$ or $R^6$, respectively) must be alkyl of 1 to 4 carbon atoms. Thus it is further provided:

(1) when $R^1$ is $OR^4$, $SR^5$, or

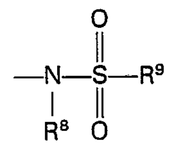

$R_2$ is other than alkyl or phenyl (unsubstituted) and $R^3$ is alkyl or 1 to 4 carbon atoms, and

(2) when $R^1$ is

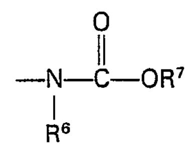

(a) $R^2$ is other than alkyl or phenyl (unsubstituted) and $R^3$ is alkyl of 1 to 4 carbon atoms; or (b) $R^7$ is other than alkyl or phenyl and $R^6$ is alkyl of 1 to 4 carbon atoms. Accordingly, when $R^2$ is other than alkyl or phenyl (unsubstituted) and $R^3$ is alkyl of 1 to 4 carbon atoms the structure of $R^6$ and $R^7$ may vary widely within the definition given above. Similarly, where $R^7$ is other than alkyl or phenyl and $R^6$ is alkyl of 1 to 4 carbon atoms the structure of $R^2$ and $R^3$ may be varied in like fashion. It will thus be understood by those skilled in the art that the present invention embraces and is applicable to the N-sulfinyl derivatives of virtually all known insecticidal carbamates, including, for example, those known by the common names carbofuran, carbaryl, aminocarb, bufencarb, butacarb, dioxacarb, ethiofencarb, methiocarb, isoprocarb, promecarb, propoxur, bendiocarb, aldicarb, methomyl, thiofanox, oxamyl, mexacarbate and others well known to those skilled in the art, including molecules containing any two such carbamates linked together by the sulfinyl group.

The N-chlorosulfinylcarbamates (II) of the invention, compounds of formula I in which $R^1$ is chloro, are prepared by reacting a carbamate starting material with thionyl chloride according to the general reaction scheme:

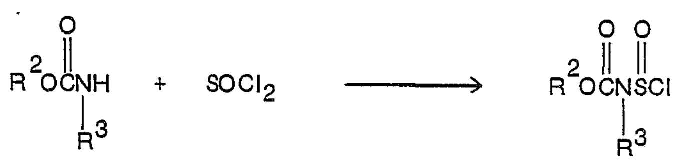

II

The reaction may be run without solvent with heating to form the corresponding N-chlorosulfinyl derivative and HCl. This reaction is generally carried out at a temperature ranging from about 60 to about 100°C. Excess thionyl chloride, is generally employed, and up to about a 50% molar excess is desirable. Hydrogen chloride is evolved during the reaction and excess thionyl chloride is removed.

The reaction between the carbamate starting material and thionyl chloride may also be carried out in a solvent, suitably a polar non-hydroxylic or non-polar organic solvent which will not react with thionyl chloride. Examples of suitable solvents are tetrahydrofuran, dichloromethane and hexane.

An acid or hydrogen chloride acceptor is added to aid the reaction. A preferred acid acceptor is pyridine, but other tertiary organic amines can be employed as acid acceptors, including dimethylaniline or diethylaniline, as well as numerous other acid acceptors well known to those skilled in the art. The reaction employing an organic solvent and acid acceptor can be carried out from room temperature up to an elevated temperature of about 60°C.

When employing an organic solvent and acid acceptor, stoichiometric molar amounts of thionyl chloride and carbamate are employed. However, if desired a small molar excess of thionyl chloride, for example up to about 10% molar excess based on the carbamate, can be employed. Approximately equimolar amounts of acid acceptor and carbamate starting material generally are employed. However,

a molar excess of acid acceptor, for example up to about at a 25% molar excess, may be employed if desired.

Examples 1 and 2 illustrate preparation of N-chlorosulfinylcarbamate esters (II) according to the invention.

Example 1

Synthesis of 2,3-dihydro-2,2-dimethylbenzofuranyl-7 N-chlorosulfinyl-N-methylcarbamate.

Five and one-half grams of carbofuran (2,3-dihydro-2,2-dimethylbenzofuranyl-7-N-methylcarbamate) (0.025 mol) were dissolved in 25 ml dry tetrahydrofuran. To this solution was added 2.5 g pyridine (0.032 mol) followed by 3.5 g thionyl chloride (0.03 mol). The mixture was stirred at room temperature for 6 hours, then filtered under nitrogen to remove pyridine hydrochloride. The solvent was evaporated under vacuum, and the viscous solution was subjected to high vacuum (0.05 mm) for 1 hour. NMR of the residue in chloroform-d-TMS showed the following absorptions: $\delta$ 7.3—6.7 (m, 3H, $\delta$ 3.05 (2, 2H, benzylic $CH_2$), $\delta$ 1.45 (s, 6H, gem-di$CH_3$).

Example 2

Synthesis of 3-isopropylphenyl N-chlorosulfinyl-N-methylcarbamate.

4 g 3-isopropylphenyl-N-methylcarbamate was mixed with an excess of thionyl chloride and heated up to 80°C until HCl evolution ceased. Excess of thionyl chloride was removed by distillation and the residue was subjected to high vacuum (0.1 mm) for 1 hour. NMR of the residue in chloroform-d-TMS gave the following absorptions: $\delta$ 7.5—6.9 (m, 4H, aromatic protons), $\delta$ 3.25 (s, 3H, $NCH_3$), $\delta$ 3.2—2.7 (m, 1H, CH), and $\delta$ 1.3—1.2 (d, 6H, $C(CH_3)_2$).

The following compounds further illustrate the N-chlorosulfinylcarbamates of this invention:

| Example | Compound |
|---|---|
| 3 | Isopropyl N-chlorosulfinyl-N-phenyl-carbamate |
| 4 | 1-Naphthyl N-chlorosulfinyl-N-methyl-carbamate |
| 5 | 2-Isopropoxyphenyl N-chlorosulfinyl-N-methyl-carbamate |
| 6 | S-Methyl-N-[N'-chlorosulfinyl-N'-methyl carbamoyloxy]-thioacetimidate |
| 7 | 2-Methyl-2-methylthio-propionaldehyde O-[N'-chlorosulfinyl-N'-methyl-carbamoyl]oxime |

These N-chlorosulfinylcarbamates are useful intermediates for preparing the pesticides of this invention, but the N-halosulfinylcarbamates are not themselves pesticides. As shown in the preceding examples these compounds may be prepared and isolated, in which case they may then be reacted with nucleophilic reactants such as alcohols, phenols, thiols, amines, carbamates, amides, sulfonamides, ureas, and sulfonyl ureas, and the like, forming the pesticides of this invention. It is preferable, however, to form the sulfinylcarbamate in place by reaction of the carbamate and thionyl chloride, then to react it without isolation with the appropriate substrate. The resulting products are effective pesticides for agricultural crop and household pests having insecticidal, nematicidal, miticidal, larvicidal and acaricidal activity, depending upon the structure of the particular carbamate ester and the functional groups.

The examples below illustrate conversion in place (without isolation) of the N-chlorosulfinylcarbamate (II) of the invention to carbamate compounds useful as pesticides in accordance with the following general reactions:

(A) II + $R^4OH$

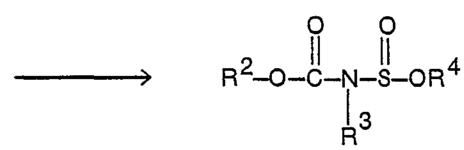

III

(B) II + $R^5SH$

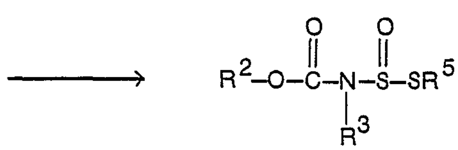

IV

(C)

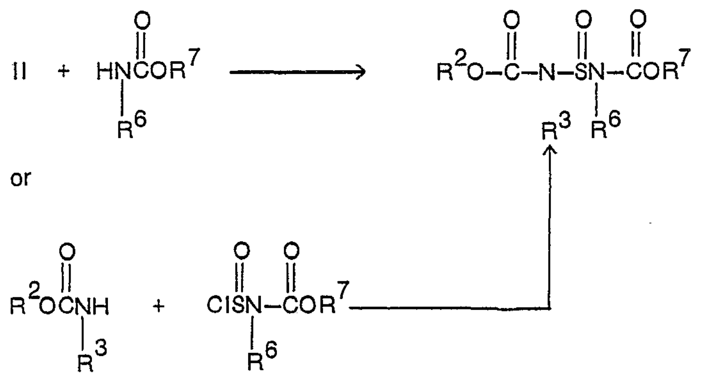

or

V

(D)

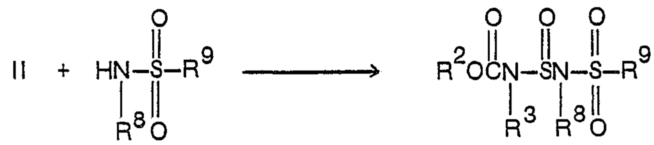

VI

Without isolation, the N-chlorosulfinylcarbamate ester reacts in place with the reactants mentioned above in the presence of an equimolar amount or molar excess of an acid or hydrogen chloride acceptor, to form the pesticidal N-sulfinylcarbamate esters, III, IV, V, or VI. The temperature of the reaction medium varies according to the reactivity of the particular reactant, and reaction system employed, but in general, can be carried out at temperatures from about 0°C to 70°C, preferably 10°C to 60°C.

Examples 8 and 9 illustrate preparation of N-(alkoxy- and aryloxy-)sulfinylcarbamates (III); that is, compounds of formula I in which $R^1$ is —$OR^4$, by in place reaction of the corresponding N-chlorosulfinylcarbamate (II) with an appropriate alcohol or phenol in accordance with general reaction scheme (A).

Example 8

Synthesis of 2,3-dihydro-2,2-dimethylbenzofuranyl-7 N-methyl-N-hexoxysulfonylcarbamate

A mixture of 2,3-dihydro-2,2-dimethylbenzofuranyl-7-N-methylcarbamate (5.5 g, 0.025 mol), pyridine (2.5 g, 0.032 mol), thionyl chloride (3.0 g, 0.025 mol), and 25 ml anhydrous tetrahydrofuran, was stirred at room temperature for 6 hours. Pyridine hydrochloride separated immediately after mixing, and stirring was continued to insure complete reaction. To this mixture was added 2 g pyridine (0.025 mol) followed by 3 g hexyl alcohol (0.029 mol) added dropwise. After stirring for an additional hour, ether (150 ml) was added to the reaction mixture. The mixture was washed with water three (30 ml each) times. The ether solution was dried over anhydrous sodium sulfate, filtered and concentrated under vacuum. Unreacted carbamate (about 0.5 g) crystallized out and was filtered. The remaining liquid, 7.4 g, was almost free from unreacted carbamate as evident from the NMR spectrum of this crude product.

A sample of this product was further purified by preparative thin layer chromatography using etherhexane (3:1) mixture as the developing solvent.

Analysis calculated for $C_{18}H_{27}O_5NS$; Carbon, 58.51%. Hydrogen, 7.37%. Found: Carbon, 59.24%, Hydrogen, 8.08%.

Example 9

Synthesis of 3-isopropylphenyl N-methyl-N-n-butoxysulfinylcarbamate

To a solution of 3-isopropylphenyl-N-methylcarbamate (3.5 g, 0.018 mol) in 20 ml anhydrous tetrahydrofuran was added 1.9 g pyridine (0.024 mol) and 2.5 g thionyl chloride (0.021 mol). The mixture was stirred for 12 hours at room temperature in order to insure complete conversion of the carbamate to the N-chlorosulfinyl derivative. Pyridine (2 g, 0.025 mol) was added, followed by 2 g n-butanol (0.027 mol), added dropwise. The mixture was stirred for an additional hour at room temperature and worked up similarly to example 8. A sample was purified by thin layer chromatography as previously described.

Analysis calculated for $C_{15}H_{23}O_4NS$; Carbon, 57.48%, Hydrogen, 7.40%. Found: Carbon, 57.80%, Hydrogen, 7.21%.

Examples 10 to 22, 61, and 62, set forth in Table I, further illustrate the N-(alkoxy- and aryloxy-) sulfinylcarbamates of this invention, having the substituent group —$OR^4$.

Examples 23 and 24 illustrate preparation of N-(alkylthio- and arylthio-)sulfinylcarbamates (IV) of this invention; that is, compounds of formula I in which $R^1$ is —$SR^5$, by reacting in place the corresponding N-chlorosulfinylcarbamate (II) and an appropriate thiol, in accordance with general reaction scheme (B).

Example 23

Synthesis of 2,3-dihydro-2,2-dimethylbenzofuranyl-7-N-methyl-N-n-butylthiosulfinylcarbamate.

To a solution of 4.4 g (0.02 mol) 2,3-dihydro-2,2-dimethylbenzofuranyl-7-N-methylcarbamate in 20 ml dry tetrahydrofuran was added 1.9 g (0.024 mol) pyridine followed by 2.5 g (0.021 mol) thionyl chloride. The mixture was stirred at room temperature for 4 hours. Pyridine (1.9 g, 0.024 mol) was added, and the mixture was cooled in an ice-water bath. To this stirring solution was added, dropwise, 1-butanethiol (1.8 g, 0.02 mol) in 2 ml tetrahydrofuran. After the complete addition of the thiol, the mixture was allowed to warm up to room temperature, and was kept at this temperature for ½ hour. Ether, 150 ml, was added, and the mixture was washed four times (25 ml each) with water. The ether solution was dried over anhydrous sodium sulfate and the solvent was evaporated under vacuum. The residue was subjected to high vacuum (0.1 mm) for several hours. NMR spectrum of this crude product showed a complete conversion of 2,3-dihydro-2,2-dimethylbenzofuranyl-7 methylcarbamate to its butylthiosulfinyl derivative. An analytical sample was obtained by preparative thin-layer chromatography.

Analysis calculated for $C_{16}H_{23}NO_4S_2$; Carbon, 53.75%; Hydrogen, 6.48%. Found: Carbon, 53.97%; Hydrogen, 6.48%.

Example 24

Synthesis of 3-isopropylphenyl N-methyl-N-2-methyl-4-t-butylphenylthiosulfinyl)carbamate.

To a solution of 4.0 g 3-isopropylphenyl-N-methylcarbamate in 20 ml dry tetrahydrofuran was added 1.9 g pyridine (0.024 mol) and 2.5 g thionyl chloride (0.021 mol). The mixture was stirred at room temperature for 6 hours. Pyridine (1.9 g, 0.024 mol) was added and followed by 3.6 g of 2-methyl-4-t-butylbenzenethiol (0.02 mol) added dropwise at room temperature. The mixture was stirred for an additional hour and 150 ml ether was added. The mixture was worked up similar to example 22 and a sample of the oily product was purified by thin-layer chromatography.

Analysis calculated for $C_{22}H_{29}NO_3S_2$; Carbon, 62.97%; Hydrogen, 6.97%. Found: Carbon, 63.40%; Hydrogen, 6.89%.

Examples 25 to 36, set forth in Table I, further illustrate the N-(alkylthio- and arylthio-)sulfinylcarbamates of this invention.

Examples 37 and 38 illustrate preparation of N,N′-sulfinyldicarbamates (V) of the invention; that is, compounds of formula I wherein $R^3$ is

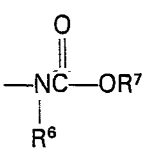

This group of compounds can be either symmetrical or asymmetrical about the sulfinyl sulfur.

In the symmetrical compounds $R^7$ above is the same as $R^2$ and $R^6$ is the same as $R^3$. These symmetrical compounds can be readily prepared in a one step process according to the following general reaction scheme

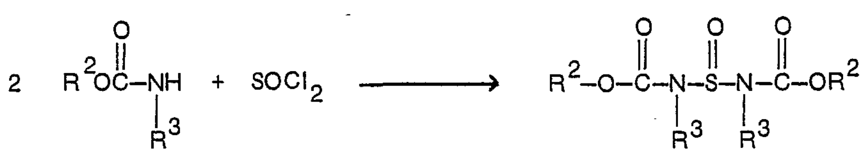

The above reaction of the carbamate ester starting material and thionyl chloride is carried out using an acid acceptor such as triethylamine in an inactive polar solvent such as tetrahydrofuran. The carbamate ester product can be formed in high yield employing two moles of the carbamate ester starting material per mole of thionyl chloride, at from 0°C to 50°C, suitably at room temperature (20°C), using about two moles or a slightly greater proportion of acid acceptor, such as triethylamine, per mole of the carbamate starting material. Example 37 illustrates this method of preparation.

Symmetrical N,N′-sulfinyldicarbamates can also be prepared by the following reaction scheme

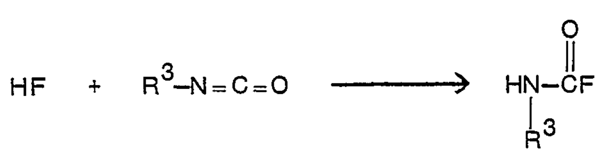

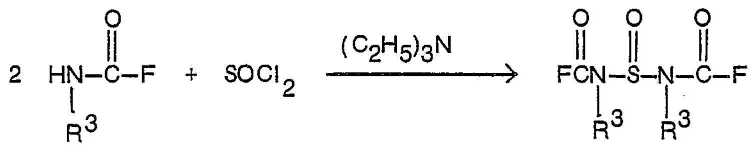


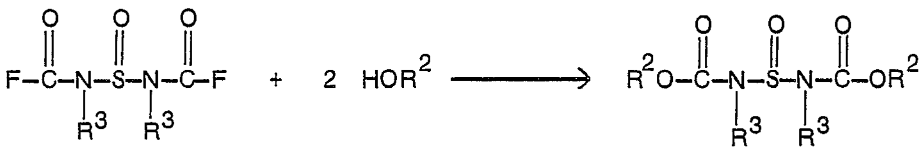

V

This procedure is less convenient as compared to the direct reaction of thionly chloride with the carbamate. However, it can be useful if $R^2$ is substituted by functional groups that are susceptible to thionyl chloride such as unsubstituted or monoalkyl substituted amides or aromatic and aliphatic amines.

Asymmetrical N,N′-sulfinyldicarbamates can be prepared by reacting N-chlorosulfinylcarbamates (II) with an alkyl, aryl, or oxime carbamate starting material in accordance with reaction scheme (C) above. Example 38 illustrates this method of preparation.

Example 37

Synthesis of N,N′-sulfinyldi-(2,3-dimethyl-2,3-dihydrobenzofuranyl-7 N-methylcarbamate).

To a solution of 2,2-dimethyl-2,3-dihydrobenzofuranyl-7-N-methylcarbamate (4.4 g, 0.02 mol) in 20 ml dry tetrahydrofuran was added 1.2 g thionyl chloride (0.01 mol). The mixture was stirred and cooled in an ice-water bath (temperature <10°C). Triethylamine (2.0 g, 0.02 mol) was added dropwise to the mixture. The temperature was allowed to rise up to room temperature after the complete addition of the amine. Stirring was continued for an additional one hour at room temperature, and 150 ml ether was added. The mixture was washed with water four times (25 ml each) and dried over anhydrous sodium sulfate. Evaporation of the solvent resulted in a solid material which was crystallized from benzene-hexane to give 3.5 g (yield 71.7%) of product, mp 139—142°.

Analysis calculated for $C_{24}H_{28}N_2O_7S$: Carbon, 59.00%; Hydrogen, 5.78%. Found: Carbon, 59.60%, Hydrogen, 5.54%.

Example 38

Synthesis of S-methyl N-[N′-(N″-ethyl-N″-n-propoxycarbonyl-aminosulfinyl)-N′-methylcarbamoyloxy] thioacetimidate.

To a solution of S-methyl N-(N′-methylcarbamoyloxy) thioacetimidate (3.3 g, 0.02 mol) in 20 ml anhydrous dichloromethane, was added 4.3 g of propyl N-ethyl-N-chlorosulfinylcarbamate (prepared from propyl ethylcarbamate and thionyl chloride, b.p. 65—69°C at 0.5 mm) and 1.6 g (0.02 mol) anhydrous pyridine. The mixture was stirred for twelve hours at room temperature. Ether (100 ml) was added and the mixture was washed with water three (30 ml each) times. The ether solution was dried over anhydrous sodium sulfate, filtered and concentrated under vacuum. A sample of the oily residue was purified by preparative thin-layer chromatography using ether-hexane (3:1) mixture as the developing solvent.

Analysis calculated for $C_{11}H_{21}N_3O_5S_2$; Carbon, 38.92%, Hydrogen, 6.23%. Found: Carbon, 38.38%; Hydrogen, 5.78%.

Examples 39 to 48, shown in Table I, further illustrate the N,N′-sulfinyldicarbamates of this invention.

Examples 49 to 51 illustrate preparation of sulfonylaminosulfinylcarbamates (VI); that is, compounds of formula I in which $R^1$ is

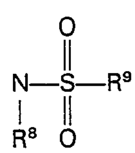

in accordance with reaction scheme (D) above.

Example 49

Synthesis of 2,3-dihydro-2,2-dimethylbenzofuranyl-7 N-(N′-benzenesulfonyl-N′-methylaminosulfinyl)-N-methylcarbamate.

To a solution of 2,3-dihydro-2,2-dimethylbenzofuranyl-7-N-methylcarbamate (4.4 g, 0.02 mol) in 15 ml tetrahydrofuran, was added pyridine (2.0 g, 0.025 mol) and thionyl chloride (2.5 g, 0.021 mol). The mixture was stirred for 4 hours at room temperature.

The reaction mixture was filtered rapidly from pyridine hydrochloride. N-Methylbenzenesulfonamide (3.5 g, 0.02 mol) was added and the mixture was cooled in an ice water bath. Triethylamine (2.2 g, 0.022 mol) was added dropwise while stirring. The mixture was stirred at room temperature for an additional half hour and diluted with 100 ml ether. The ether solution was washed with water three times (30 ml each) and then dried over anhydrous sodium sulfate.

Ether was evaporated under vacuum and the residue was subjected to high vacuum for 2 hours. A sample of the product was purified by thin layer chromatography using ethyl acetate-hexane mixture (1:3) as the developing solvent.

NMR of the purified product in chloroform-d-TMS showed the following absorptions: $\delta$ 8.0—6.6 (m, 8H, aryl protons), $\delta$ 3.0 (s, 2H, benzylic $CH_2$), $\delta$ 2.9 and $\delta$ 2.85 (two singlets, 3H each, $CONCH_3$ and $SO_2NCH_3$), $\delta$ 1.5 (s, 6H, gem-di $CH_3$).

Example 50

Synthesis of 2,3-dihydro-2,2-dimethylbenzofuranyl-7 N-(N′-methanesulfonyl-N′-ethylaminosulfinyl)-N-methylcarbamate.

To a solution of 2,3-dihydro-2,2-dimethylbenzofuranyl-7-N-methylcarbamate (4.4 g, 0.02 mol) in 15 ml tetrahydrofuran was added pyridine (2.0 g, 0.025 mol) and thionyl chloride (2.5 g, 0.021 mol). The mixture was stirred at room temperature for 4 hours. Pyridine (2.0 g, 0.025 mol) was added and followed by N-ethylmethanesulfonamide (2.7 g, 0.022 mol) and stirring was continued overnight.

Workup and purification of the final product were carried out similarly to Example 49. NMR of the purified product in chloroform-d-TMS showed the following absorptions: $\delta$ 7.1—6.7 (m, 3H aromatic protons), $\delta$ 3.75—3.55 (g, 2H, $NCH_2$), $\delta$ 3.15 (s, 6H, $CONCH_3$ and $SO_2CH_3$), $\delta$ 3.05 (s, 2H, benzylic $CH_2$), $\delta$ 1.5 (s, 6H, gem-di $CH_3$), $\delta$ 1.45—1.2 (t, 3H, $CH_3$).

Example 51

Synthesis of 2,3-dihydro-2,2-dimethylbenzofuranyl-7 N-(N′-dibutylaminosulfonyl-N′-methylaminosulfinyl)-N-methylcarbamate.

Methylisocyanate (24.0 g, 0.42 mol) was added dropwise to a cooled mixture of 40 gm 20% fuming sulfuric acid in 100 ml nitromethane. After the complete addition of the isocyanate, the resulting suspension was refluxed for 10 minutes. The mixture was filtered and the collected crystalline methylsulfamic acid was washed with ether and air dried to give 34.5 g of the dry acid (74% yield).

Methylsulfamic acid (33 g, 0.3 mol) was mixed with 62.5 g $PCl_5$ in 100 ml benzene. The mixture was heated gently until gas evolution occurred. The heat source was removed and the reaction continued spontaneously at room temperature. After most of the solids were dissolved the mixture was refluxed with stirring for a half hour. Evaporation of the solvent and distillation of the residue under vacuum gave 32 g of methylsulfamyl chloride b.p. 90—92°C./6.5 mm (82% yield).

Methylsulfamyl chloride (7.2 g, 0.056 mol) was dissolved in 75 ml dry benzene. Dibutylamine (15 g, 0.116 mol) was added dropwise at 10°C to the solution and the mixture was stirred at room temperature for 2 hours. The mixture was washed with water twice and dried over anhydrous calcium chloride. The solvent was evaporated and the residue was distilled under vacuum, to give 7.0 g of product b.p. 145—146°C/0.1 mm, which was identified as N-methyl-N′,N′-dibutylsulfondiamide by NMR spectroscopy.

2,3-Dihydro-2,2-dimethylbenzofuranyl-7-N-methylcarbamate (4.4 g, 0.02 mol) was dissolved in 20 ml dry tetrahydrofuran. To this solution was added pyridine (2.0 g, 0.025 mol) followed by thionyl chloride (2.5 g, 0.021 mol). The mixture was stirred at room temperature for 6 hours. Then pyridine (2.0 g, 0.025 mol) was added and followed by N-methyl-N′,N′-dibutylsulfondiamide (5.0 g, 0.023 mol). The mixture was stirred at room temperature overnight and ether (150 ml) was added. The mixture was washed with water three times (50 ml each) and the organic layer was dried over anhydrous sodium sulfate. The solvent was evaporated under vacuum and the residue was crystallized from hexane, m.p. 75—76°C.

NMR of the product in chloroform-d-TMS showed the following absorptions: $\delta$ 7.15—6.7 (m, 3H, aromatic protons), $\delta$ 3.35—3.1 (t, 4H, $2NCH_2$), $\delta$ 3.1 (s, 3H, $CONCH_3$), $\delta$ 3.05 (s, 2H, benzylic $CH_2$), $\delta$ 2.9 (s, 3H, $SO_2NCH_3$), $\delta$ 1.8—1.1 (m, 8H, alkyl $4CH_2$), $\delta$ 1.5 (s, 6H, gem-di$CH_3$), $\delta$ 1.1—0.85 (t, 6H, alkyl $2CH_3$).

Examples 52 to 60, set forth in Table I, further illustrate the sulfonylaminosulfinyl carbamates of this invention.

The insecticidal compounds of the invention may be formulated with the usual carriers, including additives and extenders used in the preparation of the insecticidal compositions. Thus, the toxicants of this invention, like most insecticidal agents, are generally not applied full strength, but are incorporated with the adjuvants and carriers normally employed for facilitating the dispersion of active ingredients, recognizing the accepted fact that the formulation and mode of application of a toxicant may affect the activity of the material.

The present compounds may be made into liquid concentrates by solution or emulsification in suitable liquids such as organic solvents, and into solid concentrates by admixing with talc, clays and other known solid carriers used in the insecticide art. These concentrates are compositions containing

about 5 to about 95% toxicant and the rest inert material which includes dispersing agents, emulsifying agents, and wetting agents. The concentrates are diluted for practical application, with water or other liquid for liquid sprays or with additional solid carrier for application as a dust or granular formulation.

The concentration of the toxicant in the dilution generally used for application is normally in the range of about 10% down to about 0.001% depending on the means available for application. Many variations of spraying and dusting compositions in the art may be used, by substituting a compound of this invention into the compositions known or apparent to the art.

The present compounds may also be made into composition which may be applied without further dilution, for example dusts, powders and granules. These ready to use formulations generally contain from about 0.1% to 50% of the toxicant, preferably about 1.0 to about 45%.

The insecticidal compositions may be formulated and applied with other active ingredients, including other insecticides, nematicides, acaricides, fungicides, plant regulators, fertilizers, etc. In applying the chemicals, it is obvious that an effective amount and concentration of the carbamate ester compounds of the invention should be employed.

Representative compounds of the invention were tested for insecticidal activity against two insect species, house flies, Musca domestica, and mosquito larvae, Culex pipiens. Stock 1% concentrated solutions of each of the test compounds were made in acetone, and such solutions diluted with acetone to a concentration of 0.001—0.1%. House flies were treated topically on the notum by 1 $\mu$l of each of the diluted acetone solutions. Following application, the insects were held at a constant temperature of 60°F. Percent mortality was counted 24 hours after application.

Larvicidal activity was determined by applying 1 ml of the acetone solutions in 100 ml of water containing 20 3rd instar mosquito larvae. Results are presented as $LD_{50}$ in $\mu$g/g for house flies and $LC_{50}$ in ppm for mosquito larvae.

Mammalian toxicity was determined against Swiss white mice. The test compound was administered orally using corn oil as the carrier. Results are given as $LD_{50}$ in mg of compound per kg body weight.

The test data for N-sulfinylcarbamates of the invention are summarized in Table II. In that table the term "$LD_{50}$" represents the dose needed to kill 50% of the test animals, and the term "$LC_{50}$" is the concentration needed to kill 50% of the mosquito larvae. In interpreting the values in the table, the lower the value for $LD_{50}$ for house flies and for $LC_{50}$ for mosquito larvae, the greater is the insecticidal potency or toxicity of that particular compound. On the other hand, the higher the value of $LD_{50}$ for mice, the lower the mammalian toxicity or the greater is the mammalian safety of such compound.

Referring now to Table II, the parent carbamate ester material of the compounds of Examples 8, 10—13, 23, 25—28, 37, 43—46, 49—51, 58 and 59, carbofuran, (2,3-dihydro-2,2-dimethyl-7-benzofuranyl methylcarbamate) has an $LD_{50}$ for house flies of about 6.5 and an $LD_{50}$ for mice in the range of about 2 to about 8 mg/kg. In Table II it is shown that while the corresponding compounds of this invention have a relatively comparable $LD_{50}$ for house flies (insecticidal activity), ranging from 2.2 to 20 $\mu$g/g, all have much higher $LD_{50}$ values for mice, ranging from 40 to 280, evidencing substantially lower mammalian toxicity and thus a higher margin of safety.

Similarly the other compounds of this invention retain insecticidal activity relatively comparable to that of the carbamate ester on which they are based but have substantially reduced mammalian toxicity and thus substantially greater mammalian safety.

TABLE I

| Example Number | Compound Name |
|---|---|
| 10 | 2,3-Dihydro-2,2-dimethylbenzofuranyl-7 N-methyl-N-t-butoxysulfinyl-carbamate |
| 11 | 2,3-Dihydro-2,2-dimethylbenzofuranyl-7 N-methyl-N-n-butoxysulfinyl-carbamate |
| 12 | 2,3-Dihydro-2,2-dimethylbenzofuranyl-7 N-methyl-N-phenoxysulfinyl-carbamate |
| 13 | 2,3-Dihydro-2,2-dimethylbenzofuranyl-7 N-methyl-N-2,6-dimethylphenoxysulfinyl-carbamate |
| 14 | 3-Isopropylphenyl N-methyl-N-n-hexoxysulfinyl-carbamate |
| 15 | 2-Isopropoxyphenyl N-methyl-N-n-hexoxysulfinyl-carbamate |
| 16 | 2-Isopropoxyphenyl N-methyl-N-t-butoxysulfinyl-carbamate |
| 17 | S-Methyl N-[N'-methyl-N'-hexoxysulfinylcarbamoyloxy]-thioacetimidate |

TABLE I (Continued)

| Example Number | Compound Name |
|---|---|
| 18 | S-Methyl N-[N′-methyl-N′-butoxysulfinylcarbamoyloxy]-thioacetimidate |
| 19 | 2-Methyl-2-methylthio-propionaldehyde O-[N′-methyl-N′-deoxysulfinyl-carbamoyl]-oxime |
| 20 | 1-Naphthyl N-methyl-N-hexoxysulfinyl carbamate |
| 21 | 2,2-dimethyl-1,3-benzodioxolyl-4 N-methyl-N-n-butoxysulfinyl carbamate |
| 22 | S-methyl N′,N′-dimethyl-N-*n*-butoxysulfinyl-N-methyl carbamoyloxy-1-thio-oximidate |
| 25 | 2,3-Dihydro-2,2-dimethylbenzofuranyl-7 N-methyl-N-t-butylthiosulfinyl-carbamate |
| 26 | 2,3-Dihydro-2,2-dimethylbenzofuranyl-7 N-methyl-N-phenylthiosulfinyl-carbamate |
| 27 | 2,3-Dihydro-2,2-dimethylbenzofuranyl-7 N-methyl-N-4-t-butylphenylthiosulfinyl-carbamate |
| 28 | 2,3-Dihydro-2,2-dimethylbenzofuranyl-7 N-methyl-N-2-methyl-4-t-butylphenylthio-sulfinyl-carbamate |
| 29 | 2-Isopropoxyphenyl N-methyl-N-n-butylthiosulfinyl-carbamate |
| 30 | 2-Isopropoxyphenyl N-methyl-N-2-methyl-4-t-butylphenylthiosulfinyl-carbamate |
| 31 | S-Methyl N-[N′-methyl-N′-t-butylthiosulfinyl-carbamoyloxy]thioacetimidate |
| 32 | S-Methyl N-[N′-methyl-N′-phenylthiosulfinyl-carbamoyloxy]thioacetimidate |
| 33 | 3-Isopropylphenyl N-methyl-N-t-butylthiosulfinylcarbamate |
| 34 | 2-Methyl-2-methylthio-propionaldehyde O-[N′-methyl-N-dodecylthiosulfinyl-carbamoyl]oxime |
| 35 | 1-Naphthyl N-methyl-N-t-butylthiosulfinyl carbamate |
| 36 | 1-Naphthyl N-methyl-N-phenylthiosulfinyl carbamate |
| 39 | N,N′-Sulfinyldi-(2-isopropoxyphenyl N-methylcarbamate) |
| 40 | N,N′-Sulfinyldi-[S-methyl N″-(methylcarbamoyloxy)-thioacetimidate]. |
| 41 | N,N′-Sulfinyldi-[2-methyl-2-methylthio-propionaldehyde O-(methylcarbamoyl)oxime] |
| 42 | S-Methyl N-[N′-(N″-methyl-N″-(n-propoxycarbonylaminosulfinyl)-N′-methyl-carbamoyloxy]thioacetimidate |
| 43 | 2,3-Dihydro-2,2-dimethylbenzofuranyl-7 N-[N′-methoxycarbonyl-N′-methyl-amino-sulfinyl]-N-methylcarbamate |
| 44 | 2,3-Dihydro-2,2-dimethylbenzofuranyl-7 N-[N′-ethoxycarbonyl-N′-methyl-amino-sulfinyl]-N-methylcarbamate |
| 45 | 2,3-Dihydro-2,2-dimethylbenzofuranyl-7 N-[N′-*n*-propoxycarbonyl-N′-methyl-aminosulfinyl]-N-methylcarbamate |

TABLE I (Continued)

| Example Number | Compound Name |
|---|---|
| 46 | 2,3-Dihydro-2,2-dimethylbenzofuranyl-7 N-[N′-heptoxycarbonyl-N′-methyl-aminosulfinyl]-N-methylcarbamate |
| 47 | 2-Isopropoxyphenyl N-[N′-n-propoxycarbonyl-N′-methyl-aminosulfinyl]-N-methylcarbamate |
| 48 | 3-Isopropylphenyl N-[N′-methoxycarbonyl-N′-methyl-aminosulfinyl]-N-methyl carbamate |
| 52 | 2-Isopropoxyphenyl N-(N′-benzenesulfonyl-N′-methyl-aminosulfinyl)-N-methylcarbamate |
| 53 | 3-Isopropylphenyl N-(N′-benzenesulfonyl-N′-methyl-aminosulfinyl)-N-methylcarbamate |
| 54 | 1-Naphthyl N-(N′-benzenesulfonyl-N′-methyl-aminosulfinyl)-N-methylcarbamate |
| 55 | S-Methyl N-[N′-(N″-benzenesulfonyl-N″-methyl-aminosulfinyl)-N′-methylcarbamoyloxy]thioacetimidate |
| 56 | 3-Isopropylphenyl N-(N′-methanesulfonyl-N′-ethyl-aminosulfinyl)-N-methylcarbamate |
| 57 | S-Methyl N-[N′-(N″-methanesulfonyl-N″-ethylamino-sulfinyl)-N′-methylcarbamoyloxy]thioacetimidate |
| 58 | 2,3-Dihydro-2,2-dimethylbenzofuranyl-7 N-[N′-(N″-morpholinosulfonyl)-N′-methylaminosulfinyl] N-methylcarbamate |
| 59 | 2,3-Dihydro-2,2-dimethylbenzofuranyl-7 N-(N′-morpholinosulfonyl-N′-ethyl-aminosulfinyl)-N-methylcarbamate |
| 60 | S-Methyl N-[N′-(N″-dibutylaminosulfonyl-N″-methylaminosulfinyl)-N′-methylcarbamoyloxy]thioacetimidate |
| 61 | 2,3-dihydro-2,2-dimethylbenzofuranyl-7 N-methyl-N-ethynylmethoxysulfinyl carbamate |
| 62 | 3-*i*-propylphenyl N-methyl-N-ethynylmethoxysulfinyl carbamate |

PHYSICAL DATA OF COMPOUNDS IN

TABLE I

Physical Properties

| EX. No. | Elemental Calcd (%) | Analysis Found% | Melting Pt. (°C) or Boiling Pt. (°C/mm Hg) | NMR Spectrum* (ppm $CDCl_3$) |
|---|---|---|---|---|
| 10 | C 56.28<br>H 6.79 | C 56.74<br>H 6.32 | purified by TLC | 7.0—6.8 (m, 3H)<br>1.48 (s, 6H)<br>3.0 (2s, 5H)<br>1.50 (s, 9H) |
| 11 | C 56.28<br>H 6.79 | C 56.21<br>H 6.94 | purified by TLC | 7.0—6.8 (m, 3H)<br>3.05 (2s, 5H)<br>4.1—3.9 (m, 2H)<br>1.6—1.0 (m, 3H) |
| 12 | C 59.82<br>H 5.3 | C 61.19<br>H 5.38 | purified by TLC | 7.1—6.8 (m, 8H)<br>2.92 (s, 3H)<br>3.02 (s, 2H)<br>1.5 (s, 6H) |
| 13 | C 61.68<br>H 5.95 | C 62.02<br>H 6.29 | purified by TLC | 7.1—6.8 (m, 6H)<br>2.37 (s, 2H)<br>3.24 (s, 3H)<br>1.45 (s, 6H)<br>3.05 (s, 2H) |
| 14 | C 59.8<br>H 7.97 | C 60.43<br>H 8.52 | purified by TLC | 7.2—6.8 (m, 4H)<br>3.1 (2s, 4H)<br>4.1—3.9 (m, 2H)<br>1.4—0.8 (m, 17H) |
| 15 | C 57.13<br>H 7.62 | C 57.43<br>H 7.99 | purified by TLC | 7.2—6.9 (m, 4H)<br>4.2—3.9 (m, 2H)<br>4.7—4.4 (q, 1H)<br>3.0 (s, 3H)<br>1.5—0.9 (m, 17H) |
| 16 | C 54.69<br>H 7.04 | C 55.16<br>H 7.00 | purified by TLC | 7.2—6.8 (m, 4H)<br>2.5—2.2 (m, 15H)<br>4.7—4.4 (q, 1H)<br>3.0 (s, 3H) |
| 17 | C 42.58<br>H 7.15 | C 43.62<br>H 7.67 | purified by TLC | 4.0—3.8 (m, 2H)<br>2.3 (s, 3H)<br>3.0 (s, 3H)<br>1.5—0.9 (m, 11H)<br>2.40 (s, 3H) |
| 18 | C 38.28<br>H 6.42 | C 38.71<br>H 6.57 | mp 52—54°C | 4.2—3.9 (m, 2H)<br>2.4 (s, 3H)<br>3.0 (s, 3H)<br>2.3 (s, 3H)<br>1.7—0.9 (m, 7H) |
| 19 | C 51.74<br>H 8.69 | C 51.99<br>H 8.55 | purified by TLC | 7.5 (s, 1H)<br>2.0 (s, 3H)<br>4.0—3.8 (m, 2H)<br>1.5—0.9 (m, 25H)<br>2.95 (s, 3H) |

TABLE (Continued)

Physical Properties

| EX. No. | Elemental Calcd (%) | Analysis Found% | Melting Pt. (°C) or Boiling Pt. (°C/mm Hg) | NMR Spectrum* (ppm $CDCl_3$) |
|---|---|---|---|---|
| 20 | C 61.87<br>H 6.63 | C 61.94<br>H 6.65 | purified by TLC | 8.0—7.3 (m, 7H)<br>3.15 (s, 3H)<br>4.15—3.9 (m, 2H)<br>1.8—0.8 (m, 11H) |
| 21 | | | purified by TLC | 6.8—6.5 (m, 3H)<br>3.0 (s, 3H)<br>4.1—3.85 (m, 2H)<br>1.7—0.8 (m, 13H) |
| 22 | | | purified by TLC | 4.1—3.7 (m, 2H)<br>2.33 (s, 3H)<br>3.1—2.9 (m, 9H)<br>1.7—0.8 (m, 7H) |
| 25 | C 53.75<br>H 6.48 | C 54.12<br>H 6.51 | purified by TLC | 7.15—6.8 (m, 3H)<br>1.6 (s, 9H)<br>3.25 (s, 3H)<br>1.45 (s, 6H)<br>3.05 (s, 2H) |
| 26 | C 57.27<br>H 5.07 | C 57.70<br>H 4.76 | purified by TLC | 7.7—6.7 (m, 8H)<br>1.4 (s, 6H)<br>3.2 (s, 3H)<br>3.0 (s, 2H) |
| 27 | C 60.94<br>H 6.28 | C 61.31<br>H 6.08 | purified by TLC | 7.1—6.8 (m, 3H)<br>1.6 (s, 9H)<br>3.27(s, 3H)<br>1.46 (s, 6H)<br>3.02 (s, 2H) |
| 28 | C 61.72<br>H 6.53 | C 61.97<br>H 6.74 | purified by TLC | 7.8—6.8 (m, 7H)<br>1.5 (s, 6H)<br>3.2 (s, 3H)<br>1.32 (s, 9H)<br>3.08 (s, 2H) |
| 29 | C 52.14<br>H 6.71 | C 52.80<br>H 6.79 | purified by TLC | not taken |
| 30 | C 60.66<br>H 6.71 | C 61.15<br>H 6.71 | purified by TLC | not taken |
| 31 | C 36.22<br>H 6.08 | C 36.80<br>H 6.29 | mp 51—53°C | not taken |
| 32 | | | | 7.8—7.25 (m, 5H)<br>3.25 (s, 3H)<br>2.4 (s, 3H)<br>2.25 (s, 3H) |
| 33 | C 54.68<br>H 7.04 | C 54.55<br>H 6.83 | purified by TLC | not taken |
| 34 | C 52.01<br>H 8.73 | C 52.59<br>H 9.01 | purified by TLC | not taken |

TABLE (Continued)

Physical Properties

| EX. No. | Elemental Calcd (%) | Analysis Found% | Melting Pt. (°C) or Boiling Pt. (°C/mm Hg) | NMR Spectrum* (ppm $CDCl_3$) |
|---|---|---|---|---|
| 35 | C 56.95<br>H 5.68 | C 57.51<br>H 6.82 | purified by TLC | not taken |
| 36 | | | | 8.1—6.9 (m, 12H)<br>3.2 (s, 3H) |
| 39 | C 56.88<br>H 6.08 | C 57.31<br>H 6.00 | mp 71—74°C | 7.4—6.8 (m, 8H)<br>1.3—1.2 (d, 12H)<br>4.8—4.3 (m, 2H)<br>3.1 (s, 6H) |
| 40 | C 32.45<br>H 4.90 | C 32.86<br>H 5.02 | mp 142—143°C | 3.0 (s, 6H)<br>2.23 (s, 6H)<br>2.35 (s, 6H) |
| 41 | C 39.42<br>H 6.14 | C 39.84<br>H 5.96 | mp 61—63°C | 7.65 (s, 2H)<br>1.5 (s, 12H)<br>3.0 (s, 6H)<br>2.0 (s, 6H) |
| 42 | C 36.91<br>H 5.89 | C 37.65<br>H 6.47 | purified by column chromatography | 4.34—4.1 (t, 2H)<br>3.35 (s, 3H)<br>3.0—2.95 (2s, 6H)<br>1.9—1.6 (m, 2H)<br>3.47 (s, 3H)<br>1.16—0.85 (t, 2H) |
| 43 | C 50.55<br>H 5.66 | C 51.16<br>H 5.84 | purified by TLC | 7.1—6.7 (m, 3H)<br>1.23 (s, 6H)<br>3.56 (s, 3H)<br>2.9—2.7 (m, 8H) |
| 44 | C 51.88<br>H 5.89 | C 52.33<br>H 6.98 | | not taken |
| 45 | C 53.10<br>H 6.29 | C 53.15<br>H 6.29 | | not taken |
| 46 | C 57.25<br>H 7.32 | C 57.56<br>H 7.32 | | not taken |
| 47 | C 51.55<br>H 6.44 | C 51.11<br>H 6.98 | | not taken |
| 48 | C 51.20<br>H 6.14 | C 52.06<br>H 6.14 | | not taken |
| 52 | | | | 8.1—6.8 (m, 9H)<br>4.7—4.35 (m, 1H)<br>3, 2.85 (2s, 3H ea.)<br>1.35—1.25 (d, 6H) |
| 53 | | | | 8.1—6.9 (m, 9H)<br>3, 2.9 (2s, 3H ea.)<br>3.2—2.7 (m, 1H)<br>1.3—1.2 (d, 6H) |

TABLE (Continued)

Physical Properties

| EX. No. | Elemental Calcd (%) | Analysis Found% | Melting Pt. (°C) or Boiling Pt. (°C/mm Hg) | NMR Spectrum* (ppm $CDCl_3$) |
|---|---|---|---|---|
| 54 | | | | 8.2—7.3 (m, 12H)<br>3.1, 2.9 (2s, 3H ea.) |
| 55 | | | mp 79—82°C | 8.2—7.5 (m, 5H)<br>2.95, 2.9 (2s, 3H ea.)<br>2.45 (s, 3H)<br>2.3 (s, 3H) |
| 56 | | | | 7.4—6.9 (m, 4H)<br>3.7—3.45 (q, 2H)<br>3.1 (s, 6H)<br>3.2—2.7 (m, 1H)<br>1.5—1.1 (m, 9H) |
| 57 | | | mp 125—127°C | 3.7—3.45 (q, 2H)<br>3.2 (s, 3H)<br>3.05 (s, 3H)<br>2.45 (s, 3H)<br>2.3 (s, 3H)<br>1.4—1.2 (t, 3H) |
| 58 | | | | 7.2—6.7 (m, 3H)<br>3.9—3.7 (m, 4H)<br>3.6—3.0 (m, 4H)<br>3.1 (s, 3H)<br>3.05 (s, 2H)<br>3.0 (s, 3H)<br>1.45 (s, 6H) |
| 59 | | | | 7.2—6.7 (m, 3H)<br>3.9—3.6 (m, 4H)<br>3.6—3.0 (m, 6H)<br>3.1 (s, 3H)<br>3.0 (s, 2H)<br>1.5 (s, 6H)<br>1.5—1.3 (m, 3H) |
| 60 | | | mp 61—63°C | 3.35—3.15 (t, 4H)<br>3.0 (s, 3H)<br>2.9 (s, 3H)<br>2.4 (s, 3H)<br>2.3 (s, 3H)<br>1.8—1.1 (m, 8H)<br>1.05—0.75 (t, 6H) |
| 61 | | | | 6.9—6.5 (m, 3H)<br>2.85 (s, 2H)<br>4.5 (m, 2H)<br>1.33 (s, 6H)<br>2.9 (s, 3H) |

TABLE (Continued)

Physical Properties

| EX. No. | Elemental Calcd (%) | Analysis Found% | Melting Pt. (°C) or Boiling Pt. (°C/mm Hg) | NMR Spectrum* (ppm $CDCl_3$) |
|---|---|---|---|---|
| 62 | | | | 7.4—6.8 (m, 4H)<br>3.95—3.7 (m, 1H)<br>4.66—4.5 (m, 2H)<br>2.55—2.45 (m, 1H)<br>3.1 (s, 3H)<br>1.3—1.2 (d, 6H) |

*s = singlet
d = doublet
t = triplet
m = multiplet
q = quartet

TABLE II

| Compound of Example | House Flies $LD_{50}$ (μg/g) | Culex $LC_{50}$ (ppm) | Mice $LD_{50}$ (mg/kg) |
|---|---|---|---|
| 8 | 13 | 0.0025 | 280 |
| 9 | 50 | — | — |
| 10 | 2.2 | 0.0058 | 40 |
| 11 | 16 | — | 160 |
| 12 | 6.4 | 0.0055 | 42 |
| 13 | 13.5 | — | 100 |
| 14 | 80 | — | >1000 |
| 15 | 42 | 0.01 | >1000 |
| 16 | 65 | — | 400 |
| 17 | 6.5 | — | 260 |
| 18 | 5.5 | — | — |
| 19 | 11.5 | 0.006 | 60 |
| 20 | 360 | — | >1000 |
| 23 | 16.5 | — | 45 |
| 24 | 62.5 | — | 450 |
| 25 | 9 | 0.01 | 70 |
| 26 | 7 | 0.05 | 32 |
| 27 | 11.8 | — | 92 |

TABLE II (Continued)

| Compound of Example | House Flies $LD_{50}$ ($\mu g/g$) | Culex $LC_{50}$ (ppm) | Mice $LD_{50}$ (mg/kg) |
|---|---|---|---|
| 28 | 8.8 | — | 70 |
| 29 | 31 | — | 1050 |
| 30 | 27 | — | >1000 |
| 31 | 5.5 | — | 135 |
| 32 | 6.5 | — | 150 |
| 33 | 75 | — | 250 |
| 34 | 12 | — | — |
| 35 | 235 | — | >1000 |
| 36 | 350 | — | >1000 |
| 37 | 20 | — | 165 |
| 38 | 7.0 | — | 115 |
| 39 | 85 | — | 940 |
| 40 | 40 | — | 210 |
| 41 | 6.5 | — | 1.8 |
| 42 | 6.6 | — | 160 |
| 43 | 9.0 | — | 62 |
| 44 | 13.0 | 0.04 | 56 |
| 45 | 9.0 | — | 100 |
| 46 | 13.5 | 0.015 | 150 |
| 47 | 20 | 0.18 | 500—1000 |
| 48 | 80 | — | 70 |
| 49 | 9.5 | — | 35 |
| 50 | 12.5 | — | 75 |
| 51 | 12 | — | 25 |
| 52 | 55 | — | 250 |
| 53 | 95 | — | 150 |
| 54 | 450 | — | >500 |
| 55 | 17.5 | — | 86 |
| 56 | 90 | — | 200 |

TABLE II (Continued)

| Compound of Example | House Flies $LD_{50}$ ($\mu$g/g) | Culex $LC_{50}$ (ppm) | Mice $LD_{50}$ (mg/kg) |
|---|---|---|---|
| 57 | 25 | — | 250 |
| 58 | 13 | — | — |
| 59 | 10 | — | — |
| 60 | 9 | — | 100 |

## Claims

1. A compound of the formula

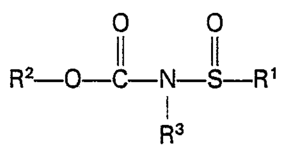

characterized in that

$R^1$ is a halogen atom selected from bromine, chlorine and fluorine;

$—OR^4$, $—SR^5$, 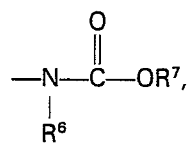 or 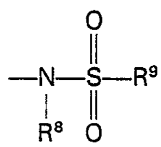

$R^2$ and $R^7$, the same or different, are alkyl of 1 to 8 carbon atoms; naphthyl; phenyl; an aryl group of the formula

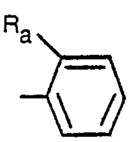 or 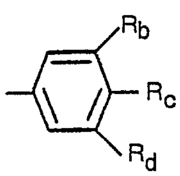

wherein

$R_a$ is alkyl, alkoxy, alkylthio, alkylthioalkyl or 2-dioxolanyl,

$R_b$ is hydrogen, alkyl, alkoxy, alkoxyalkyl, formyl(alkyl)amino, or halogen,

$R_c$ is hydrogen, alkyl, alkoxy, alkylthio or dialkylamino,

$R_d$ is hydrogen or alkyl, and each alkyl of $R_a$, $R_b$, $R_c$ or $R_d$ group contains 1 to 6 carbon atoms; a heterocyclic group of the formula

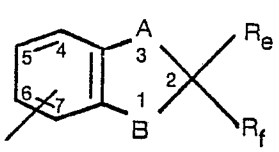

in which $R_e$ and $R_f$ are hydrogen or alkyl of 1 to 4 carbon atoms, A and B are each oxygen, or one of A and B is methylene and the other is oxygen or sulfur, attached at the 4 or 7 position; or an imino group of the formula $R_gR_hC{=}N—$ wherein $R_g$ is hydrogen or alkyl of 1 to 4 carbon atoms and $R_h$ is alkylthio, alkylthioalkyl or dialkylaminocarbonyl in which the alkyl groups, the same or different, have 1 to 4 carbon atoms;

$R^3$, $R^6$ and $R^8$, the same or different, are alkyl of 1 to 4 carbon atoms or phenyl optionally substituted with alkyl of 1 to 4 carbon atoms or halogen;

$R^4$ and $R^5$ are alkyl of 1 to 12 carbon atoms; an unsaturated aliphatic hydrocarbon of 2 to 4 carbon atoms; or phenyl optionally substituted with alkyl of 1 to 6 carbon atoms, alkylthio of 1 to 4 carbons or di($C_{1-4}$)alkylamino;

$R^9$ is alkyl of 1 to 4 carbon atoms; phenyl optionally substituted with alkyl or halogen; or the group —$NR^{10}R^{11}$, in which $R^{10}$ and $R^{11}$ are alkyl of 1 to 6 carbons or taken together with the nitrogen form a morpholino, piperidyl, or pyridyl ring; with the provisos:

(1) when $R^1$ is —$OR^4$, —$SR^5$, or

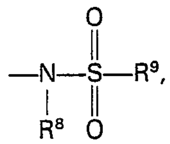

$R^2$ is other than alkyl or phenyl (unsubstituted) and $R^3$ is alkyl of 1 to 4 carbon atoms; and

(2) when $R^1$ is

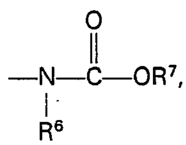

(a) $R^2$ is other than alkyl or phenyl (unsubstituted) and $R^3$ is alkyl of 1—4 carbon atoms; or (b) $R^7$ is other than alkyl or phenyl (unsubstituted) and $R^6$ is alkyl of 1 to 4 carbon atoms.

2. The compound of claim 1 characterized in that $R^1$ is —$OR^4$.

3. The compound of claim 1 characterized in that $R^1$ is —$SR^5$.

4. The compound of claim 1 characterized in that $R^1$ is

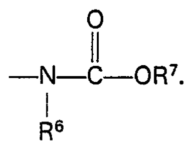

5. The compound of claim 4 characterized in that $R^7$ is the same as $R^2$ and $R^6$ is the same as $R^3$.

6. The compound of claim 4 characterized in that $R^7$ and $R^2$ are different.

7. The compound of claim 1 characterized in that $R^1$ is

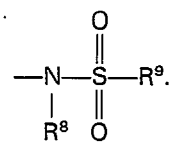

8. The compound of claim 1 characterized in that at least one of $R^2$ and $R^7$ is a heterocyclic group of the formula

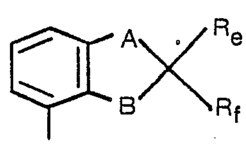

in which $R_e$ and $R_f$ are hydrogen or alkyl of 1 to 4 carbon atoms, A and B are oxygen or one of A and B is methylene and the other is oxygen or sulfur.

9. The compound of claim 8 characterized in that at least one of $R^2$ and $R^7$ is a 2,3-dihydro-2,2-dimethyl-7-benzofuranyl group and at least one $R^3$ and $R^6$ is methyl.

10. The compound of claim 1 characterized in that at least one of $R^2$ and $R^7$ is naphthyl or a substituted aryl group of the formula

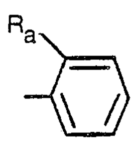

or

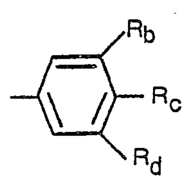

11. The compound of claim 1 characterized in that at least one of $R^2$ and $R^7$ is an imino group of the formula $R_gR_hC{=}N{-}$.

12. The compound of claim 1 characterized in that $R^1$ is chloro.

13. An insecticidal composition characterized in containing an insecticidally effective amount of a compound of any one of claims 1, 2, 3, 4, 5, 6, 7, 8, 9, 10 and 11 other than a compound in which $R^1$ is a halogen atom, in admixture with an agriculturally acceptable adjuvant or carrier.

14. A method for control of insects in an agricultural crop characterized in applying to the crop or to the soil in which the crop is planted or is to be planted an insecticidally effective amount of a compound of any one of claims 1, 2, 3, 4, 5, 6, 7, 8, 9, 10 and 11 other than a compound in which $R^1$ is a halogen atom.

15. A process for preparing the compound of claim 12 characterized in reacting a carbamate of the formula

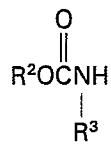

with an equimolar amount or molar excess of thionyl chloride.

16. The process of claim 15 characterized in that said reaction is conducted without solvent with heating.

17. The process of claim 15 characterized in that said reaction is conducted in the presence of a polar non-hydroxylic or non-polar organic solvent and an acid acceptor.

18. A process for preparing the compound of claim 2, 3, 4, 6, 7, 8, 9, 10 or 11 characterized in reacting the compound of claim 12 with a compound of the formula

$R^4OH$, $R^5SH$, 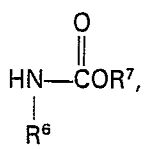 , or 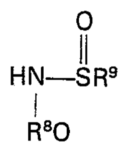

19. The process of claim 18 characterized in that the reaction is conducted in the presence of a non-hydroxylic polar solvent or of a non-polar organic solvent in the presence of an acceptor.

20. The process of claim 19 characterized in that the compound of claim 12 is formed in place and reacted without isolation, with a compound of the formula

$R^4OH$, $R^5SH$, 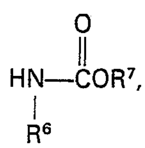 , or 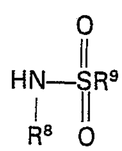

21. The process for preparing a compound of claim 5 characterized in reacting of a carbamate of the formula

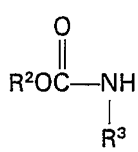

with thionyl chloride in the presence of an acid acceptor and an inactive polar solvent, about 2 mols of said carbamate being employed per mol of thionyl chloride.

## Patentansprüche

1. Eine Verbindung der Formel

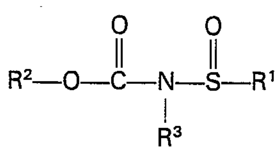

dadurch gekennzeichnet, daß

$R^1$ ein Brom-, Chlor- oder Fluoratom oder die Reste

$—OR^4$, $—SR^5$, 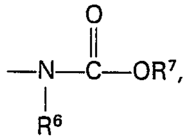 oder 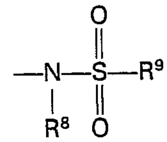

bedeutet,

$R^2$ und $R^7$ gleich oder verschieden sind, einen Alkylrest mit 1 bis 8 Kohlenstoffatomen, eine Naphthyl- oder Phenylgruppe, einen Arylrest der Formeln

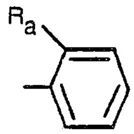 oder 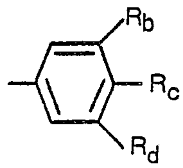

in denen

$R_a$ ein Alkyl-, Alkoxy-, Alkylthio-, Alkylthioalkyl- oder 2-Dioxolanylrest ist,

$R_b$ ein Wasserstoffatom, ein Alkyl-, Alkoxy-, Alkoxyalkyl- oder Formyl(alkyl)aminorest oder ein Halogen ist,

$R_c$ ein Wasserstoffatom oder ein Alkyl-, Alkoxy-, Alkylthio oder Dialkylaminorest ist,

$R_d$ ein Wasserstoffatom oder ein Alkylrest ist, und die Alkylgruppe in den $R_a$, $R_b$, $R_c$ oder $R_d$ Resten jeweils 1 bis 6 Kohlenstoffatome enthält, bedeuten, einen heterocyclischen Rest der Formel

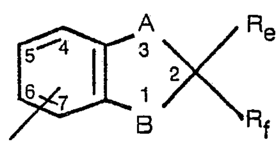

bedeuten, in der $R_e$ und $R_f$ ein Wasserstoffatom oder ein Alkylrest mit 1 bis 4 Kohlenstoffatomen ist, A und B je ein Sauerstoffatom bedeuten oder einer der Reste A oder B eine Methylengruppe und der andere Rest ein Sauerstoffatom oder ein Schwefelatom bedeutet, das an die 4- oder 7-Stellung gebunden ist, oder

eine Iminogruppe der Formel $R_gR_hC{=}N—$ bedeuten, wobei $R_g$ ein Wasserstoffatom oder ein Alkylrest mit 1 bis 4 Kohlenstoffatomen ist und $R_h$ ein Alkylthio-, Alkylthioalkyl- oder Dialkylaminocarbonylrest ist und die Alkylreste gleich oder verschieden sind und 1 bis 4 Kohlenstoffatomen haben,

$R^3$, $R^6$ und $R^8$ gleich oder verschieden sind und einen Alkylrest mit 1 bis 4 Kohlenstoffatomen oder eine gegebenenfalls mit einem Alkylrest mit 1 bis 4 Kohlenstoffatomen oder durch Halogen substituierte Phenylgruppe bedeuten,

$R^4$ und $R^5$ einen Alkylrest mit 1 bis 12 Kohlenstoffatomen, einen ungesättigten aliphatischen Kohlenwasserstoffrest mit 2 bis 4 Kohlenstoffatomen oder eine gegebenenfalls mit einem Alkylrest mit 1 bis 6 Kohlenstoffatomen, einem Alkylthiorest mit 1 bis 4 Kohlenstoffatomen oder mit einem $Di(C_{1-4})$-alkylaminorest substituierten Phenylgruppe bedeuten,

$R^9$ einen Alkylrest mit 1 bis 4 Kohlenstoffatomen, eine gegebenenfalls mit einem Alkylrest oder einem Halogenatom substituierten Phenylgruppe oder den Rest $—NR^{10}R^{11}$ bedeutet, wobei $R^{10}$ und $R^{11}$ einen Alkylrest mit 1 bis 6 Kohlenstoffatomen bedeuten oder zusammen mit dem Stickstoffatom einen Morpholino-, Piperidyl- oder Pyridylring bilden,

mit der folgenden Maßgabe:

(1) wenn $R^1$ $—OR^4$, $—SR^5$ oder

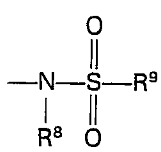 ist,

bedeutet $R^2$ keinen Alkyl- oder unsubstituierten Phenylrest und $R^3$ stellt einen Alkylrest mit 1 bis 4 Kohlenstoffatomen dar, und

(2) wenn $R^1$

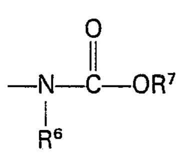 ist,

(a) stellt $R^2$ keinen Alkyl- oder unsubstituierten Phenylrest dar und $R^3$ bedeutet einen Alkylrest mit 1 bis 4 Kohlenstoffatomen; oder (b) stellt $R^7$ keinen Alkyl- oder unsubstituierten Phenylrest dar und $R^6$ bedeutet einen Alkylrest mit 1 bis 4 Kohlenstoffatomen.

2. Die Verbindung nach Anspruch 1, dadurch gekennzeichnet, daß $R^1$ den Rest $—OR^4$ bedeutet.

3. Die Verbindung nach Anspruch 1, dadurch gekennzeichnet, daß $R^1$ den Rest $—SR^5$ bedeutet.

4. Die Verbindung nach Anspruch 1, dadurch gekennzeichnet, daß $R^1$ den Rest

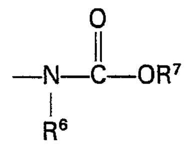

bedeutet.

5. Die Verbindung nach Anspruch 4, dadurch gekennzeichnet, daß $R^7$ dieselbe Bedeutung wie $R^2$ und $R^6$ dieselbe Bedeutung wie $R^3$ hat.

6. Die Verbindung nach Anspruch 4, dadurch gekennzeichnet, daß $R^7$ und $R^2$ verschieden sind.

7. Die Verbindung nach Anspruch 1, dadurch gekennzeichnet, daß $R^1$ den Rest

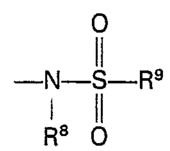

bedeutet.

8. Die Verbindung nach Anspruch 1, dadurch gekennzeichnet, daß mindestens einer der Reste $R^2$ und $R^7$ einen heterocyclischen Rest der Formel

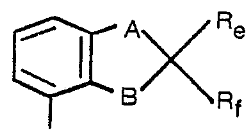

bedeutet, in der $R_e$ und $R_f$ ein Wasserstoffatom oder einen Alkylrest mit 1 bis 4 Kohlenstoffatomen bedeuten und A und B Sauerstoffatome sind oder einer der Reste A und B eine Methylengruppe und der andere ein Sauerstoff- oder Schwefelatom bedeutet.

9. Die Verbindung nach Anspruch 8, dadurch gekennzeichnet, daß mindestens einer der Reste $R^2$ und $R^7$ eine 2,3-Dihydro-2,2-dimethyl-7-benzofuranylruppe bedeutet und mindestens einer der Reste $R^3$ und $R^6$ eine Methylgruppe ist.

10. Die Verbindung nach Anspruch 1, dadurch gekennzeichnet, daß mindestens einer der Reste $R^2$ und $R^7$ eine Naphthylgruppe oder ein substituierter Arylrest der Formeln

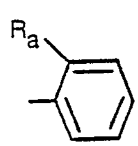

oder

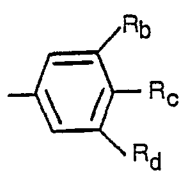

bedeutet.

11. Die Verbindung nach Anspruch 1, dadurch gekennzeichnet, daß mindestens einer der Reste $R^2$ und $R^7$ eine Iminogruppe der Formel $R_gH_hC{=}N—$ bedeutet.

12. Die Verbindung nach Anspruch 1, dadurch gekennzeichnet, daß $R^1$ ein Chloratom ist.

13. Ein insektizides Mittel, gekennzeichnet durch einen Gehalt einer insektizid wirksamen Menge einer Verbindung nach einem der Ansprüche 1, 2, 3, 4, 5, 6, 7, 8, 9, 10 oder 11, wobei $R^1$ kein Halogenatom bedeutet, im Gemisch mit einem in der Landwirtschaft anwendbaren Hilfsmittel oder Trägerstoff.

14. Ein Verfahren zur bekämpfung von Insekten bei Nutzpflanzen, dadurch gekennzeichnet, daß man eine insektizid wirksame Menge einer Verbindung von einem der Ansprüche 1, 2, 3, 4, 5, 6, 7, 8, 9, 10 oder 11, wobei $R^1$ kein Halogenatom bedeutet, auf die Nutzpflanze oder den Boden, in dem die Feldfrüchte gepflanzt sind oder werden, aufbringt.

15. Ein Verfahren zur Herstellung der Verbindung nach Anspruch 12, dadurch gekennzeichnet, daß man ein Carbamat der Formel

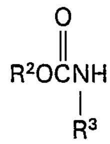

mit einer äquimolaren Menge oder einem molaren Überschuß an Thionylchlorid umsetzt.

16. Das Verfahren nach Anspruch 15, dadurch gekennzeichnet, daß man die Umsetzung in Abwesenheit eines Lösungsmittels unter Erhitzen durchführt.

17. Das Verfahren nach Anspruch 15, dadurch gekennzeichnet, daß man die Umsetzung in Gegenwart eines polaren oder nicht-polaren organischen Lösungsmittels, das keine Hydroxylgruppen enthält, und einem Säureakzeptor durchführt.

18. Ein Verfahren zur Herstellung der Verbindung nach Anspruch 2, 3, 4, 6, 7, 8, 9, 10 oder 11, dadurch gekennzeichnet, daß man die Verbindung gemäß Anspruch 12 mit einer Verbindung der Formeln

$R^4OH$, $R^5SH$, 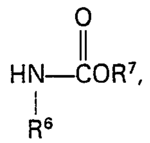, oder 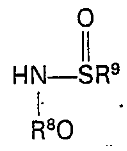

umsetzt.

19. Das Verfahren nach Anspruch 18, dadurch gekennzeichnet, daß man die Umsetzung in Gegenwart eines polaren Lösungsmittels, das keine Hydroxylgruppen enthält oder eines nicht-polaren organischen Lösungsmittels in Gegenwart eines Akzeptors durchführt.

20. Das Verfahren nach Anspruch 19, dadurch gekennzeichnet, daß die Verbindung gemäß Anspruch 12 an Ort und Stelle gebildet und ohne Isolierung weiter umgesetzt wird mit einer Verbindung der Formeln

$R^4OH$, $R^5SH$, 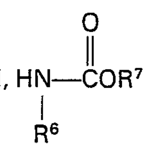 oder 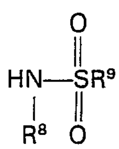

21. Ein Verfahren zur Herstellung einer Verbindung nach Anspruch 5, dadurch gekennzeichnet, daß man ein Carbamat der Formel

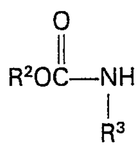

mit Thionylchlorid in Gegenwart eines Säureakzeptors und eines inaktiven polaren Lösungsmittels umsetzt, wobei etwa 2 Mol des Carbamats pro Mol Thionylchlorid eingesetzt werden.

**Revendications**

1. Composé représenté par la formule ci-après:

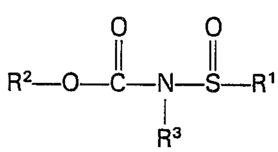

caractérisé en ce que:

$R^1$ est un atome d'halogène choisi parmi les atomes de brome, chlore ou fluor; un radical

$—OR^4$, $—SR^5$, 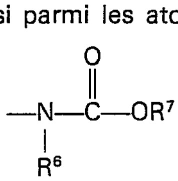 ou 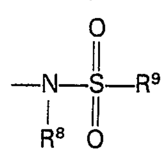

$R^2$ et $R^7$, identiques ou différents, sont les groupes alcoyle de 1 à 8 atomes de carbone; naphtyle; phényle; un groupe aryle représenté par les formules:

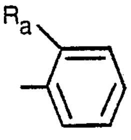

ou

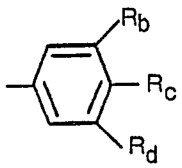

où:

$R_a$ est un radical alcoyle, alcoxy, alcoylthio, alcoylthioalcoyle ou 2-dioxolanyle,

$R_b$ est un atome d'hydrogène, un groupe alcoyle, alcoxy, alcoxyalcoyle, formyl(alcoyl)amino ou un atome d'halogène,

$R_c$ est un atome d'hydrogène, un groupe alcoyle, alcoxy, alcoylthio ou dialcoylamino,

$R_d$ est un atome d'hydrogène ou un groupe alcoyle, et chacun des groupes alcoyle des radicaux $R_a$, $R_b$, $R_c$ ou $R_d$ contient 1 à 6 atomes de carbone;
un groupe hétérocyclique représenté par la formule:

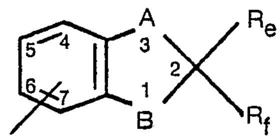

dans laquelle $R_e$ et $R_f$ sont un atome d'hydrogène ou un groupe alcoyle de 1 à 4 atomes de carbone, A et B sont chacun un atome d'oxygène, ou bien l'un des radicaux A et B est un groupe méthylène et l'autre un atome d'oxygène ou de soufre, fixé en position 4 ou 7; ou
un groupe imino représenté par la formule $R_gR_hC{=}N$— dans laquelle $R_g$ est un atome d'hydrogène ou un groupe alcoyle de 1 à 4 atomes de carbone, et $R_h$ est un groupe alcoylthio, alcoylthioalcoyle ou dialcoylaminocarbonyle dans lequel les groupes alcoyle, identiques ou différents, ont 1 à 4 atomes de carbone;

$R^3$, $R^6$ et $R^8$, identiques ou différents, sont des groupes alcoyle de 1 à 4 atomes de carbone ou phényle éventuellement substitués par un groupe alcoyle de 1 à 4 atomes de carbone ou un atome d'halogène;

$R^4$ et $R^5$ sont des groupes alcoyle de 1 à 12 atomes de carbone; un hydrocarbure aliphatique insaturé de 2 à 4 atomes de carbone; ou un groupe phényle éventuellement substitué par un groupe alcoyle de 1 à 6 atomes de carbone, alcoylthio de 1 à 4 atomes de carbone ou di($C_{-4}$)alcoylamino;

$R^9$ est un groupe alcoyle de 1 à 4 atomes de carbone; phényle éventuellement substitué avec un groupe alcoyle ou un atome d'halogène; ou le groupe —$NR^{10}R^{11}$, dans lequel $R^{10}$ et $R^{11}$ sont des groupes alcoyle de 1 à 6 atomes de carbone ou pris ensemble avec l'atome d'azote, ils forments un cycle morpholino, pipéridyle, ou pyridyle; à condition que:

(1) lorsque $R^1$ est —$OR^4$, —$SR^5$, ou

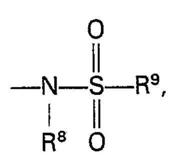

$R^2$ est autre qu'un radical alcoyle ou phényl non substitué et $R^3$ est un groupe alcoyle de 1 à 4 atomes de carbone; et

(2) lorsque $R^1$ est le radical

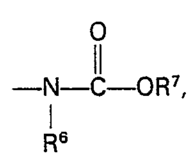

(a) $R^2$ est autre qu'un radical alcoyle ou phényle non substitué et $R^3$ est un groupe alcoyle de 1 à 4 atomes de carbone; ou (b) $R^7$ est autre qu'un radical alcoyle ou phényle non substitué et $R^6$ est un groupe alcoyle de 1 à 4 atomes de carbone;

2. Composé selon la revendication 1, caractérisé en ce que $R^1$ est —$OR^4$.

3. Composé selon la revendication 1, caractérisé en ce que $R^1$ est —$SR^5$.

4. Composé selon la revendication 1, caractérisé en ce que $R^1$ est

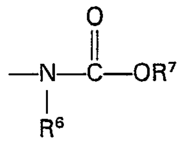

5. Composé selon la revendication 4, caractérisé en ce que $R^7$ est le même que $R^2$ et $R^6$ est le même que $R^3$.

6. Composé selon la revendication 4, caractérisé en ce que $R^7$ et $R^2$ sont différents.

7. Composé selon la revendication 1, caractérisé en ce que $R^1$ est

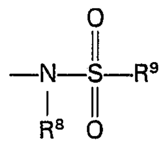

8. Composé selon la revendication 1, caractérisé en ce que l'un au moins des groupes $R^2$ et $R^7$ est un groupe hétérocyclique représenté par la formule:

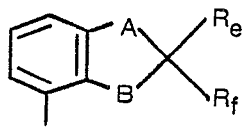

dans laquelle $R_e$ et $R_f$ sont un atome d'hydrogène ou un groupe alcoyle de 1 à 4 atomes de carbone, A et B sont un atome d'oxygène ou l'un des groupes A et B est un groupe méthylène et l'autre est un atome d'oxygène ou de soufre.

9. Composé selon la revendication 8, caractérisé en ce que l'un au moins des groupes $R^2$ et $R^7$ est un groupe 2,3-dihydro-2,2-diméthyl-7-benzofuranyle et l'un au moins des groupes $R^3$ et $R^6$ est un radical méthyle.

10. Composé selon la revendication 1, caractérisé en ce que l'un au moins des groupes $R^2$ et $R^7$ est un groupe naphtyle ou un groupe aryle substitué représenté par la formule:

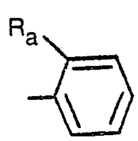

ou

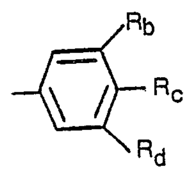

11. Composé selon la revendication 1, caractérisé en ce que l'un au moins des groupes $R^2$ et $R^7$ est un groupe imino représenté par la formule $R_gR_hC{=}N{-}$.

12. Composé selon la revendication 1, caractérisé en ce que $R^1$ est un atome de chlore.

13. Composition insecticide caractérisée en ce qu'elle contient une quantité efficace du point de vue insecticide d'un composé selon l'une quelconque des revendications 1, 2, 3, 4, 5, 6, 7, 8, 9, 10 et 11, autre qu'un composé dans lequel $R^1$ est un atome d'halogène, en mélange avec un support ou adjuvant acceptable du point de vue agricole.

14. Méthode pour le contrôle des insectes dans une culture agricole, caractérisée en ce que l'on applique à cette culture ou au sol dans lequel est plantée la culture ou doit être plantée, une quantité efficace du point de vue insecticide d'un composé selon l'une quelconque des revendications 1, 2, 3, 4, 5, 6, 7, 8, 9, 10 et 11, autre qu'un composé dans lequel $R^1$ est un atome d'halogène.

15. Procédé pour préparer le composé selon la revendication 12, caractérisé en ce que l'on fait réagir un carbamate représenté par la formule:

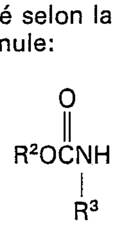

avec une quantité équimolaire ou un excès molaire de chlorure de thionyle.

16. Procédé selon la revendication 15, caractérisé en ce que cetté reaction est réalisée sans solvant, avec chauffage.

17. Procédé selon la revendication 15, caractérise en ce que cette réaction est réalisée en présence d'un solvant organique non polaire ou polaire non hydroxylique et d'un accepteur d'acide.

18. Procédé pour préparer le composé selon l'une des revendications 2, 3, 4, 6, 7, 8, 9, 10 ou 11, caractérisé en ce que l'on fait réagir le composé selon la revendication 12 avec un composé représenté par la formule:

$R^4OH$, $R^5SH$, 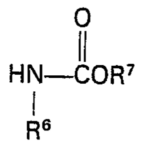 ou 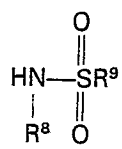

19. Procédé selon la revendication 18, caractérisé en ce que la réaction est réalisé en présence d'un solvant polaire non hydroxylique ou d'un solvant organique non polaire en présence d'un accepteur.

20. Procédé selon la revendication 19, caractérisé en ce que le composé de la revendication 12 est formé sur place et traité, sans isolation, avec un composé représenté par la formule

$R^4OH$, $R^5SH$, 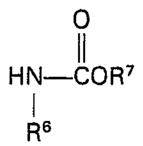 ou 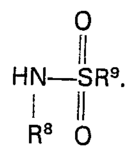

21. Procédé pour préparer un composé selon la revendication 5, caractérisé en ce que l'on fait réagir un carbamate représenté par la formule ci-après:

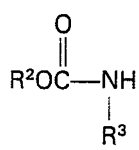

avec du chlorure de thionyle en présence d'un accepteur d'acide et dans un solvant polaire inactif, à raison d'environ 2 moles de carbamate par mole de chlorure de thionyle.